# EUROPÄISCHE PATENTSCHRIFT

(11) **EP 0 648 748 B1**
(45) Veröffentlichungstag und Bekanntmachung des Hinweises auf die Patenterteilung: **02.05.1997**
(21) Anmeldenummer: 94116235.6
(22) Anmeldetag: 14.10.1994
(51) Int. Cl.: C07D 235/26

(54) **Verfahren zur Herstellung von 5-Acetoacetylaminobenzimidazolon-2**
Process for the preparation of 5-acetoacetylaminobenzimidazolon-2
Procédé pour la préparation de 5-acétoacéthylaminobenzimidazolone-2

(30) Priorität: 19.10.1993 DE 4335614
(43) Veröffentlichungstag der Anmeldung: 19.04.1995
(73) Patentinhaber: HOECHST AKTIENGESELLSCHAFT, 65929 Frankfurt am Main (DE)
(72) Erfinder: Mack, Karl Ernst, Dr., D-65207 Wiesbaden (DE); Bohusch, Michael, Dr., D-61267 Neu Anspach (DE)

(56) Entgegenhaltungen:
- DE-A- 1 795 051
- DE-A- 2 518 922
- DE-A- 2 612 391
- DATABASE WPI Section Ch, Week 8237, Derwent Publications Ltd., London, GB; Class E14, AN 82-77578E & JP-A-57 126 453 (DAINIPPON INK & CHEM KK) 6. August 1982
- DATABASE WPI Section Ch, Week 9338, Derwent Publications Ltd., London, GB; Class A60, AN 93-299611 & JP-A-5 213 896 (HONSHU CHEM IND CO LTD) 24. August 1993

## Beschreibung

5-Acetoacetylaminobenzimidazolon-2 ist ein bedeutendes Ausgangsprodukt zur Herstellung wasserunlöslicher Monoazofarbstoffe. In DE-OS 1795051, die sich mit der Herstellung dieser Farbstoffe befaßt, wird erwähnt, daß man 5-Acetoacetylaminobenzimidazolon-2 Acetoacetylaminobenzimidazolon-2 durch Umsetzung von mit Diketen in Wasser oder organischen Lösungsmitteln wie z.B. Essigsäure erhalten kann, genauere Angaben, wie z.B. Reaktionsbedingungen sind nicht aufgeführt.

Aus GB-770263 ist bekannt, wasserunlösliche primäre aromatische Amine mit Diketen in Form ihrer in Wasser gelösten Salze von verschiedenen Säuren umzusetzen, wobei die Temperatur nicht über 50°C steigen darf. Wird nach dem dort beschriebenen Verfahren jedoch 5-Aminobenzimidazolon-2 eingesetzt, so wird die Acetoacetylverbindung nur in unzureichender Reinheit erhalten.

In DE-PS 2518922 wird vorgeschlagen, das bei Raumtemperatur in Wasser nahezu unlösliche 5-Aminobenzimidazolon-2 nach Überführung in Salze verschiedener Säuren bei 60 bis 100°C mit Diketen in wäßriger Lösung umzusetzen. Aufgrund der erhöhten Löslichkeit der Salze und der vorgeschlagenen schnellen Zugabe ("in einem Schuß") werden in diskontinuierlichen Umsetzungen zwar hohe Raum-Zeit-Leistungen erreicht, die Ausbeute an 5-Acetoacetylaminobenzimidazolon-2 ist jedoch geringer als 85 %.

Die Reaktion von gelösten primären aromatischen Aminen mit Diketen beschreibt auch die japanische Offenlegung 57-126453. Hier werden die Amine vorzugsweise in Alkohol gelöst und bei maximal 50°C durch langsame Zugabe von Diketen umgesetzt. Eine möglicherweise vorteilhafte Übertragung dieser Verfahrensweise auf die Umsetzung von 5-Acetoacetylaminobenzimidazolon-2 wird dadurch erschwert, daß bei den genannten Temperaturen nur sehr verdünnte Lösungen des Amins von ca. 2 Gew.-% in z.B. Methanol oder Ethanol eingesetzt werden können. Außerdem stehen die langen Reaktionszeiten von mindestens einer Stunde der technisch interessanten Nutzung des Verfahrens in kontinuierlicher Durchführung entgegen.

Aus DE-OS 1518881 ist bekannt, die Umsetzung von aromatischen Aminen mit Diketen in Wasser kontinuierlich bei Temperaturen bis maximal 50°C durchzuführen. Langsame Dosierung der Reaktionspartner läßt auch hier auf lange Umsetzungszeiten und auf große Apparatedimensionen schließen. Aufgrund der Schwerlöslichkeit der Amine in Wasser müssen zusätzlich Verunreinigungen der Produkte durch mitgefälltes Amin in Betracht gezogen werden. Bei der Verfahrensübertragung auf das nahezu wasserunlösliche 5-Aminobenzimidazolon-2 verstärken sich diese Probleme.

In DE-PS 2612391 wird daher vorgeschlagen, 5-Aminobenzimidazolon-2, oder auch dessen Salze ausgewählter Säuren in wäßriger Lösung mit Diketen bei Temperaturen bis etwa 90°C kontinuierlich umsetzen. Allerdings begrenzt die Löslichkeit des 5-Aminobenzimidazolon-2 in Wasser selbst bei 90°C die Konzentration der Einsatzlösungen auf etwa 4 Gew.-% und läßt dann, wenn die bei maximal 90°C konzentrierten Aminlösungen bei niedrigeren Reaktionstemperaturen von nur 80 - 85°C oder gar von 60°C (maximale Amin-Konzentration einer Lösung ca. 1,5 Gew.-%) mit Diketen umgesetzt werden, Ausfällungen des Amins erwarten, die als Verunreinigung im Produkt auftreten. Weiterhin wird in DE-PS 2612391 vorgeschlagen, die exotherme Umsetzung z.B. durch kontinuierliche Zugabe von Kaltwasser zu kühlen, wodurch allerdings eine weitere Verdünnung des Reaktionsgemisch eintritt. Dies trägt zum Produktverlust in das Abwasser des Verfahrens bei und beschränkt die Ausbeute bezüglich Amin auf ca. 90 %.

In Anbetracht dieser Einschränkungen und Nachteile bestand ein großes Bedürfnis nach einem verbesserten Verfahren, durch das die den bekannten Verfahren innewohnenden Nachteile vermieden und gute bis sehr gute Ausbeuten, hohe Reinheit und verkürzte Reaktionszeiten ermöglicht werden, das apparativ einfach durchzuführen ist und das nur zu einer geringen Abwasserbelastung führt.

Diese Aufgabe wird gelöst durch ein Verfahren zur Herstellung von 5-Acetoacetylaminobenzimidazolon-2 Acetoacetylaminobenzimidazolon-2 durch kontinuierliche Umsetzung von mit Diketen dadurch gekennzeichnet, daß die Umsetzung in Gegenwart eines wasserlöslichen (C₁-C₄)-Alkohols oder eines Gemisch dieses Alkohols mit Wasser bei Siedetemperatur erfolgt.

Die Umsetzung wird allgemein so durchgeführt, daß Diketen und eine heiß gesättigte Lösung von 5-Aminobenzimidazolon-2 getrennt, aber gleichzeitig und kontinuierlich in einen Reaktor dosiert und umgesetzt werden. Aufgrund der spontan freiwerdenden Reaktionswärme gelangt der Reaktorinhalt zum Sieden. Die Dämpfe werden kondensiert und das Kondensat z.B. zum Reaktor zurückgeführt. Das sich bildende 5-Acetoacetylaminobenzimidazolon-2 kristallisiert während der Umsetzung teilweise aus und wird in dem Maße, wie die Einsatzkomponenten zugeführt werden, als Suspension dem Reaktor entzogen. Die Isolierung des Produkts kann anschließend diskontinuierlich oder vorteilhaft kontinuierlich erfolgen. Die zum Einsatz kommende Lösung des Amins kann als Alkohol Methanol, Ethanol, einen der Propanole oder Butanole, oder aber ein Gemisch aus einem dieser Alkohole und Wasser enthalten. Im letzten Fall ist es zweckmäßig einen bei Raumtemperatur mit Wasser unbegrenzt mischbarem Alkohol wie Methanol, Ethanol, n-Propanol oder Isopropanol zu verwenden.

Der Bereich der einzelnen Siedepunkte der zweckmäßig verwendeten Alkohole beträgt ca. 65°C für Methanol und ca. 98°C für n-Propanol. Etwa den gleichen Siedebereich von ca. 65°C bis 100°C umfassen die Gemische der Alkohole mit Wasser, so daß durch geeignete Zusammensetzung jede gewünschte Siedetemperatur, d.h. Umsetzungstemperatur eingestellt werden kann. Weiterhin wird durch die Art des Alkohols und durch seinen Anteil in wäßrigem Gemisch die maximale Löslichkeit von 5-Aminobenzimidazolon-2 bestimmt. So können z.B. ca. 12 Gew.-% Amin enthaltende Lösungen aus Methanol mit 20 Gew.-% Wasser oder aber auch aus Ethanol mit 50 Gew.-% Wasser hergestellt werden, die unterschiedliche Siedepunkte von ca. 70°C bzw. von ca. 83°C aufweisen. Demgegenüber werden bei diesen Temperaturen in reinem Wasser nur Amin-Konzentrationen von ca. 1,5 Gew.-% bzw. ca. 3 Gew.-% erreicht.

Die Art des verwendeten Alkohols, der Zusatz an Wasser, die Siedepunkte d.h. die Umsetzungstemperaturen - gegebenenfalls beeinflußt durch veränderten Druck- und das Lösevermögen für das Amin stehen in komplexer Beziehung zueinander. Die Vielfalt der Variationen der Parameter erlaubt somit eine Optimierung der Umsetzungsbedingungen unter mannigfaltigen Gesichtspunkten wie z.B. bereits vorhandene technische Apparate, besondere Qualitätsanforderungen an das Produkt oder auch Berücksichtigung des Einsatzes von getrocknetem oder wasserfeuchtem Amin.

Als zweckmäßig hat sich erwiesen, die Umsetzungstemperaturen wegen der thermischen Instabilität des Diketens aus sicherheitstechnischen Gründen auf ca. 95°C zu begrenzen und um ausreichend rasche Umsetzungen zu ermöglichen, Temperaturen oberhalb von 65°C zu wählen. Es hat sich in vielen Fällen bewährt bei Temperaturen von 70°C bis 90°C zu arbeiten. Die Temperatur des Einsatzstroms des gelösten Amins sollte stets nur geringfügig unterhalb der Umsetzungstemperatur liegen, um maximale Aminkonzentrationen zu gewährleisten. Der Diketenstrom wird allgemein nicht vorgewärmt. Weiterhin ist es zweckmäßig, die Umsetzung allgemein unter Normaldruck durchzuführen. Überdruck oder Vakuum beeinflussen die Umsetzungstemperatur und sind daher im genannten Anwendungsbereich der Temperatur nur begrenzt anwendbar.

Eine vorteilhafte Ausführungsform des neuen Verfahrens ist z.B. unter Einsatz von technisch wasserfeucht verfügbarem Amin die Verwendung von Ethanol mit 45 bis 55 Gew.-%, Wasser zum Lösen des Amins und die kontinuierliche Umsetzung mit Diketen bei 83°C bis 85°C unter Sieden des Umsetzungsgemischs und Rückfluß.

Nach der Isolierung von 5-Acetoacetylaminobenzimidazolon-2 aus dem Aufarbeitungsstrom verbleibt ein Teil des Produkts entsprechend seiner Löslichkeit in der Mutterlauge. Die hochselektiv verlaufende Umsetzung nach dem neuen Verfahren erlaubt es, ohne nennenswerten Qualitätsverlust des Produkts, die Mutterlauge zur Umsetzung zurückzuführen, indem sie zum Lösen des Amins verwendet wird. Auf diese Weise kann nach der folgenden Umsetzung das in der Mutterlauge enthaltene Produkt unter deutlicher Ausbeutesteigerung weitgehend gewonnen werden. Die Rückführung der Mutterlauge stellt daher eine bevorzugte Ausführungsform des neuen Verfahrens dar.

Als Reaktoren eignen sich für die Umsetzung Apparate, in denen einerseits eine intensive Vermischung der Einsatzkomponenten möglich ist, wie z.B. durch Rührer, durch statische Mischer oder durch Düsensysteme. Andererseits soll die Verdampfungskühlung eine optimale Wärmeabfuhr und Konstanz bzw. Begrenzung der Umsetzungstemperatur für die arbeitstechnisch sichere Reaktionsführung gewährleisten. Geeignete Reaktoren sind z.B. Rührkessel oder Rührkesselkaskade, wobei gegebenenfalls zur Erhöhung der Mischwirkung die Einsatzkomponenten über statische Mischer oder Mischdüsen dosiert werden. Weiterhin können als Reaktoren typische Verdampfungsapparate wie z.B. Umlaufverdampfer, Fallfilm- oder Dünnschichtverdampfer dienen, die mit einem Mischaggregat für die Einsatzströme versehen sind. Wegen der schnellen Umsetzung von Amin und Diketen können hohe Durchsatzmengen im Reaktor von ca. 50 kg bis 150 kg Einsatzlösung pro Stunde und Liter Reaktorvolumen erreicht werden. Aufgrund der hohen Aminkonzentration von über 10 Gew.-% können ca. 10 kg bis 25 kg Produkt pro Stunde und Liter im Reaktor erzeugt werden.

Katalysatoren sind bei der Umsetzung allgemein nicht erforderlich, können aber zur weiteren Beschleunigung in bekannter Weise verwendet werden. Hierzu zählen Protonsäuren wie z.B. Essigsäure, Amine wie z.B. Triethylamin und auch Ammoniumverbindungen. Andere Zusätze wie farbaufhellende Substanzen sind allgemein nicht erforderlich, obwohl es sich beim Einsatz besonders dunkelgefärbten Amins als vorteilhafte erweist, geringe Mengen z.B. an Dithionit während der Umsetzung hinzuzufügen.

Die Aufarbeitung der kontinuierlich aus dem Reaktor ausgeschleusten Suspension erfolgt in bekannter Weise durch Kühlen zur Vervollständigung der Kristallisation von 5-Acetoacetylaminobenzimidazolon-2 und Isolierung auf einem geeigneten Filter. Die anfallende Mutterlauge kann allgemein als Lösemittel für das Einsatzamin wiederverwendet werden.

Das anfallende Rohprodukt wird allgemein zur Entfernung anhaftender Mutterlauge z.B. mit einer geringen Menge Alkohol oder wäßrigen Alkohol gewaschen, das gereinigte Produkt bei Bedarf getrocknet oder durch schonende Destillation in einer wäßrigen Suspension von Alkohol befreit und als wasserfeuchtes Produkt isoliert. Nach destillativer Rückgewinnung von wäßrigen Alkohol aus der geringe Menge angefallener Waschlauge erzeugt das neue Verfahren praktisch kein Abwasser.

Das neue Verfahren zeichnet sich aufgrund des Siedens des Umsetzungsgemischs und der damit verbundenen technisch einfach durchführbaren effektiven Siedekühlung sowie der Temperaturbegrenzung durch hohe Arbeitssicherheit aus. Weiterhin werden große Produktmengen in kleinen Reaktorvolumen wegen der geringen Reaktionszeiten und hohen Konzentrationen der Einsatzlösung des Amins von über 10 Gew.-% erzielt. Ein Merkmal des neuen Verfahrens ist es auch, daß die nach der Produktisolierung anfallende - weiterhin Produkt enthaltende - Mutterlauge geeignet ist, als Lösungsmittel für das Amin in weiteren Umsetzungscyclen wiederverwendet zu werden. Auf diese Weise gelingt es, das Verfahren - insbesondere bei der Verwendung von wäßrigen Alkoholen - praktisch abwasserfrei zu gestalten und durch weitere Produktisolierung die Ausbeute auf ca. 96 % bezüglich Amin zu steigern.

Das Beispiel soll das Verfahren erläutern, ohne es jedoch darauf zu beschränken.

### Beispiel

Eine Lösung aus 2,86 kg (19,2 Mol) 5-Aminobenzimidazolon-2 und 24 kg 50 Gew.-% Wasser enthaltendes Ethanol mit einer Temperatur von 82°C wird pro Stunde kontinuierlich und gleichzeitig mit 2,06 kg (24 Mol) Diketen (Reinheit 98 %) in einen Reaktor dosiert, der mit einem Rührer, Rührflußkühler, Thermometer und einem Ablauf am unteren Ende versehen ist. Die helle Suspension im Reaktor siedet unter Rühren und Rückfluß bei ca. 85°C und wird durch Abpumpen auf ein konstantes Volumen von ca. 0,3 l gehalten. Die ausgeschleuste Suspension wird auf ca. 15°C abgekühlt und die hellen Kristalle durch Filtrieren von stündlich 22 kg Mutterlauge abgetrennt, mit durchschnittlich 6 kg pro Stunde 50 %igem Ethanol gewaschen und getrocknet. Es werden pro Stunde 4,03 kg (17,3 Mol) 5-Acetoacetylaminobenzimidazolon-2 als nahezu weißes Kristallpulver erhalten. Die Ausbeute bezüglich Amin beträgt 90 %.

Die angefallene Mutterlauge wird in vier weiteren Umsetzungscyclen wiederverwendet, indem sie frisches 50 Gew.-% Wasser enthaltendes Ethanol zum Lösen von 2,86 kg/h 5-Aminobenzimidazolon-2 bei 80°C in dem Maß ersetzt, daß 26,86 kg/h Aminlösung in oben beschriebener Weise mit 2,06 kg/h Diketen umgesetzt werden können. Nach Aufarbeitung werden 4,25 bis 4,34 kg/h entsprechend einer Ausbeute von 95 bis 97 % bezüglich Amin an 5-Acetoacetylaminobenzimidazolon-2 gleichbleibender Qualität erhalten.

## Patentansprüche

1. Verfahren zur Herstellung von 5-Acetoacetylaminobenzimidazolon-2 durch kontinuierliche Umsetzung von 5-Amino benzimidazolon-2 mit Diketen dadurch gekennzeichnet, daß die Umsetzung in Gegenwart eines wasserlöslichen (C₁-C₄)-Alkohols oder eines Gemischs dieses Alkohols mit Wasser bei Siedetemperatur erfolgt.

2. Verfahren nach Anspruch 1, dadurch gekennzeichnet, daß als Alkohol Methanol, Ethanol, n-Propanol oder i-Propanol verwendet wird.

3. Verfahren nach Anspruch 1 oder 2, dadurch gekennzeichnet, daß der Alkohol oder das Alkohol/Wasser-Gemisch einen Siedepunkt von 65°C bis 100°C, insbesondere 70°C bis 90°C aufweist.

4. Verfahren nach Anspruch 1 oder 2, dadurch gekennzeichnet, daß Ethanol mit 45 bis 55 Gew.-%, Wasser verwendet wird.

5. Verfahren nach mindestens einem der Ansprüche 1 bis 4, dadurch gekennzeichnet, daß der Alkohol oder das Alkohol/Wasser-Gemisch in Form rückgeführter Mutterlauge eingesetzt wird.

## Claims

1. A process for preparing 5-acetoacetylamino-2-benzimidazolone by continuous reaction of 5-amino-2-benzimidazolone with diketene, which comprises carrying out the reaction in the presence of a water-soluble (C₁-C₄)-alcohol or of a mixture of this alcohol with water at the boiling temperature.

2. The process of claim 1, wherein the alcohol used is methanol, ethanol, n-propanol or isopropanol.

3. The process of claim 1 or 2, wherein the alcohol or alcohol-water mixture has a boiling point from 65°C to 100°C, in particular from 70°C to 90°C.

4. The process of claim 1 or 2, wherein ethanol containing from 45 to 55% by weight of water is used.

5. The process of at least one of claims 1 to 4, wherein the alcohol or alcohol-water mixture is used in the form of recirculated mother liquor.

## Revendications

1. Procédé de préparation de la 5-acétoacétylaminobenzimidazolone-2 par réaction en continu de la 5-aminobenzimidizolone-2 avec des dicetènes, caractérisé en ce qu'on met en oeuvre la réaction en présence d'un alcool en C₁-C₄ soluble dans l'eau, ou d'un mélange de cet alcool avec l'eau, à la température d'ébullition.

2. Procédé selon la revendication 1, caractérisé en ce qu'on utilise comme alcool le méthanol, l'éthanol, le n-propanol ou l'i-propanol.

3. Procédé selon la revendication 1 ou 2, caractérisé en ce que l'alcool ou le mélange d'alcool/eau présente un point d'ébullition de 65°C à 100°C, plus particulièrement de 70°C à 90°C.

4. Procédé selon la revendication 1 ou 2, caractérisé en ce qu'on utilise l'éthanol ayant une teneur en eau de 45 à 55% en poids.

5. Procédé selon au moins l'une des revendications 1 à 4, caractérisé en ce qu'on utilise l'alcool ou le mélange d'alcool/eau sous forme de liqueur-mère recyclée.
